(19) Europäisches Patentamt
European Patent Office
Office européen des brevets

(11) EP 1 130 100 A1

(12) EUROPEAN PATENT APPLICATION

(43) Date of publication:
**05.09.2001 Bulletin 2001/36**

(51) Int Cl.⁷: **C12N 15/55**, C12N 9/20, C12Q 1/68

(21) Application number: **01200375.2**

(22) Date of filing: **02.02.2001**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **14.02.2000 EP 00200513**

(71) Applicants:
- **UNILEVER N.V.**
  **3013 AL Rotterdam (NL)**
  Designated Contracting States:
  **BE CH DE DK ES FI FR GR IT LI LU MC NL PT SE TR AT**
- **UNILEVER PLC**
  **London EC4P 4BQ (GB)**
  Designated Contracting States:
  **GB IE**

(72) Inventors:
- **Brocca, Stefania**
  **20134 Milano (IT)**
- **Bornscheuer, Uwe T., E.-M.-Arndt University**
  **17487 Greifswald (DE)**
- **Pleiss, Jürgen**
  **70569 Stuttgart (DE)**
- **Schmid, Rolf D.**
  **70569 Stuttgart (DE)**
- **Schmid, Ulrike**
  **1521 AZ Wormerveer (NL)**
- **Schmitt, Jutta**
  **70569 Stuttgart (DE)**

(74) Representative: **Sikken, Antonius H. J. M. et al**
**UNILEVER N.V.,**
**Patent Division,**
**P.O. Box 137**
**3130 AC Vlaardingen (NL)**

# (54) Modified lipolytic enzymes and their use

(57) There is provided an amino acid sequence encoding a lipase, which has at least 65% sequence homology with the amino acid seqence of a *Candida ruginosa* lipase having the amino acid sequence substantially as depicted in Figure 1 and SEQ ID NO:2, and which differs by at least one amino acid substitution at a selected site in said lipase.

Also provided is a modified nucleic acid sequence encoding a lipase variant, preferably a variant of lipase 1 wherein the modified residues are located at one or more of the positions T11 and T201 in the amino acid sequence of *Candida rugosa* lipase 1. Preferably, the threonine at one or more of the positions 11 and 201 is replaced by alanine.

Furthermore, the use of the lipase variants and/or amino acid sequences is provided in a manner known per se, in particular in a process requiring high specificity towards $C_{16}$-$C_{18}$ acyl chains.



Printed by Jouve, 75001 PARIS (FR)

## SEQUENCE LISTING

<110> Unilever N.V.

<120> Modified lipolytic enzymes and their use

<130> F 7523 (V)

<140>
<141>

<160> 8

<170> PatentIn Ver. 2.1

<210> 1
<211> 1950
<212> DNA
<213> Artificial Sequence

<400> 1
cggatcggac tactagcagc tgtaatacga ctcactatag ggaatattaa gcttttgatt 60
ttaacgactt ttaacgacaa cttgagaaga tcaaaaaaca actaattatt cgaaacgatg 120
agatttcctt caatttttac tgctgtttta ttcgcagcat cctccgcatt agctgcccca 180
accgccactt tggctaacgg tgacaccatc accggtttga acgccatcat caacgaagcc 240
ttcttgggta ttccatttgc cgaaccacca gttggtaact tgagattcaa ggacccagtt 300
ccatactccg gttccttgga tggtcaaaag ttcacttctt acggtccatc ttgtatgcaa 360
caaaacccag aaggtaccta cgaagaaaac ttgccaaagg cagctttaga tctggttatg 420
caatccaaag ttttcgaagc tgtttctcca tcttctgaag actgtttgac cattaatgtt 480
gttagaccac ccgggacaaa ggctggtgcc aacttgccag ttatgttgtg gatctttggt 540
ggtggttttg aagttggtgg tactagtacc ttccctccag cccaaatgat taccaagtct 600
attgctatgg gtaagccaat catccacgtt tctgtcaact acagagtctc gagctggggt 660
ttcttggctg gtgacgaaat caaggccgaa ggttctgcca acgccggttt gaaggaccaa 720
agattgggta tgcaatgggt ggctgacaac attgctgctt ttggtggtga tccaactaag 780
gttactatct ttggtgaatc tgctggttct atgtccgtca tgtgtcacat tttgtggaac 840
gacggtgaca acacttacaa gggtaagcca ttgttcagag ctggtatcat gcaatctggt 900
gctatggttc catctgacgc cgtcgacggt atctacggta acgaaatttt tgacttgttg 960
gcttccaacg ctggttgtgg ttctgcctct gacaagttgg cttgtttgag aggtgtttct 1020
tctgacactt tggaagacgc caccaacaac acccctggtt tcttggctta ctcctcctta 1080
agattgtctt acttgccaag accagacggt gttaacatca ccgacgacat gtacgctttg 1140
gttagagaag gtaagtatgc caacatccct gttatcatcg gtgaccaaaa cgacgaaggt 1200
accttctttg gtacttcttc tttgaacgtt accactgatg cccaagccag agaatatttc 1260
aagcaatctt ttgtccacgc tagcgacgct gaaatcgaca ctttgatgac tgcttaccca 1320
ggtgacatca ctcaaggttc tccatttgac actggaattc taaacgcctt gaccccacaa 1380
ttcaagagaa tctctgctgt tttgggtgac ttgggtttta ctttggctcg tagatacttc 1440
ttgaaccact acaccggtgg taccaagtac tctttcttgt ctaagcaatt gtctggtttg 1500
ccagttttgg gtactttcca ctccaacgat atcgtcttcc aagactactt gttgggttct 1560
ggttccttga tctacaacaa cgctttcatt gcttttgcca ctgacttgga cccaaacacc 1620
gccggtttgt tggttaagtg gccagaatac acctcttctt ctcaatctgg taacaacttg 1680
atgatgatca acgctttggg tttgtacacc ggtaaggaca acttcagaac cgccggttac 1740
gacgctttgt tctccaaccc accatctttc tttgtttgat aataaggcct ggatccacta 1800
gtaacggccg ccagtgtgct ggaattctgc agatatccat cacactggcg gccgctcgag 1860
catgcatcta gagggccgca tcatgtaatt agttatgtca cgcttacatt cacgccctcc 1920
ccccacatcc gctctaaccg aaaaggaagg 1950

<210> 2
<211> 534

<212> PRT
<213> Candida rugosa

<400> 2

```
Ala Pro Thr Ala Thr Leu Ala Asn Gly Asp Thr Ile Thr Gly Leu Asn
1               5                   10                  15

Ala Ile Ile Asn Glu Ala Phe Leu Gly Ile Pro Phe Ala Glu Pro Pro
            20                  25                  30

Val Gly Asn Leu Arg Phe Lys Glu Pro Val Gln Tyr Ser Gly Ser Leu
        35                  40                  45

Asp Gly Gln Lys Phe Thr Ser Tyr Gly Pro Ser Cys Met Gln Gln Asn
    50                  55                  60

Pro Glu Gly Thr Tyr Glu Glu Asn Leu Pro Lys Ala Ala Leu Asp Leu
65                  70                  75                  80

Val Met Gln Ser Lys Val Phe Glu Ala Val Ser Pro Ser Ser Glu Asp
                85                  90                  95

Cys Leu Thr Ile Asn Val Val Arg Pro Pro Gly Thr Lys Ala Gly Ala
            100                 105                 110

Asn Leu Pro Val Met Leu Trp Thr Phe Gly Gly Gly Phe Glu Thr Gly
        115                 120                 125

Gly Thr Arg Thr Phe Pro Pro Ala Gln Met Ile Thr Lys Ser Ile Ala
    130                 135                 140

Met Gly Lys Pro Ile Ile His Val Ser Val Asn Tyr Arg Val Ser Ser
145                 150                 155                 160

Trp Gly Phe Leu Ala Gly Asp Glu Ile Lys Ala Glu Gly Ser Ala Asn
                165                 170                 175

Ala Gly Leu Lys Asp Gln Arg Leu Gly Met Gln Trp Val Ala Asp Asn
            180                 185                 190

Ile Ala Ala Phe Gly Gly Asp Pro Thr Lys Val Thr Ile Phe Gly Glu
        195                 200                 205

Ser Thr Thr Ser Met Ser Val Met Cys His Ile Leu Trp Asn Asp Gly
    210                 215                 220

Asp Asn Thr Tyr Lys Gly Lys Pro Leu Phe Arg Ala Gly Ile Met Gln
225                 230                 235                 240

Ser Gly Ala Met Val Pro Ser Asp Ala Val Asp Gly Ile Tyr Gly Asn
                245                 250                 255
```

Glu Ile Phe Asp Leu Leu Ala Ser Asn Ala Gly Cys Gly Ser Ala Ser
260 265 270

Asp Lys Leu Ala Cys Leu Arg Gly Val Ser Ser Glu Thr Leu Glu Asp
275 280 285

Ala Thr Asn Asn Thr Pro Gly Phe Leu Ala Tyr Ser Ser Leu Arg Leu
290 295 300

Ser Tyr Leu Pro Arg Pro Asp Gly Val Thr Ile Thr Asp Asp Met Tyr
305 310 315 320

Ala Leu Val Arg Glu Gly Lys Tyr Ala Asn Ile Pro Val Ile Ile Gly
325 330 335

Asp Gln Asn Asp Glu Gly Thr Phe Phe Gly Thr Ser Ser Leu Asn Val
340 345 350

Thr Thr Asp Ala Gln Ala Arg Glu Tyr Phe Lys Gln Ser Phe Val His
355 360 365

Ala Ser Asp Thr Glu Ile Asp Thr Leu Met Thr Ala Tyr Pro Gly Asp
370 375 380

Ile Thr Gln Gly Ser Pro Phe Asp Thr Gly Ile Leu Asn Ala Leu Thr
385 390 395 400

Pro Gln Phe Lys Arg Ile Ser Ala Val Leu Gly Asp Leu Gly Phe Thr
405 410 415

Leu Ala Arg Arg Tyr Phe Leu Asn His Tyr Thr Gly Gly Thr Lys Tyr
420 425 430

Ser Phe Leu Ser Lys Gln Leu Ser Gly Leu Pro Val Leu Gly Thr Phe
435 440 445

His Ser Asn Asp Ile Val Phe Gln Asp Tyr Leu Leu Gly Ser Gly Ser
450 455 460

Leu Ile Tyr Asn Asn Ala Phe Ile Ala Phe Ala Thr Asp Leu Asp Pro
465 470 475 480

Asn Thr Ala Gly Leu Leu Val Lys Trp Pro Glu Tyr Thr Ser Ser Ser
485 490 495

Gln Ser Gly Asn Asn Gly Met Met Ile Asn Ala Leu Gly Leu Tyr Thr
500 505 510

Gly Lys Asp Asn Phe Arg Thr Ala Gly Tyr Asp Ala Leu Phe Phe Asn
515 520 525

Pro Pro Ser Phe Phe Val
530

<210> 3
<211> 20
<212> DNA
<213> Artificial Sequence

<220>
<223> Description of Artificial Sequence: primer_bind

<400> 3
cggatcggac tactagcagc 20

<210> 4
<211> 19
<212> DNA
<213> Artificial Sequence

<220>
<223> Description of Artificial Sequence: primer_bind

<400> 4
cggttagagc ggatgtggg 19

<210> 5
<211> 18
<212> DNA
<213> Artificial Sequence

<220>
<223> Description of Artificial Sequence: primer_bind

<400> 5
gcagctgtaa tacgactc 18

<210> 6
<211> 25
<212> DNA
<213> Artificial Sequence

<220>
<223> Description of Artificial Sequence: primer_bind

<400> 6
ttagaccacc cgggacaaag gctgg 25

```
<210> 7
<211> 27
<212> DNA
<213> Artificial Sequence

<220>
<223> Description of Artificial Sequence: primer_bind

<400> 7
atctgacgcc gtcgacggta tctacgg                                   27



<210> 8
<211> 18
<212> DNA
<213> Artificial Sequence

<220>
<223> Description of Artificial Sequence: primer_bind

<400> 8
cgtgaatgta agcgtgac                                             18
```

## Description

### Technical Field

The present invention generally relates to the field of lipolytic enzymes. More in particular, the invention relates to the application of recombinant DNA technology for improving the properties of lipolytic enzymes. Specifically, the invention is concerned with novel lipases having altered substrate specificity, to methods for their preparation, and their use in biocatalytic applications.

### Background and Prior Art

Lipases (triacylglycerol lipase EC 3.1.1.3) catalyse the hydrolysis of triglycerides at water/oil interfaces. In vitro, however, they are versatile enzymes because of their ability of catalysing the hydrolysis and synthesis of a great variety of esters (i.e. the hydrolysis and transesterification of triacylglycerols), and the resolution of racemic mixtures. They are also employed in detergents (due to their ability to remove fat and ink stains), and in food industry where they are used in, for example, fat retailoring of triglycerides.

*Candida rugosa* lipases, also referred to hereinafter as "CRLs", are among the commercial lipases most often employed in hydrolysis and synthesis of a wide range of esters of commercial interest[1]. CRL is an unspecific lipase and shows main activity against medium chain fatty acids. CRL discriminates polyunsaturated fatty acids and is used for their enrichment. In most biocatalytic applications crude enzyme preparations, obtained by TCA precipitation of the culture supernatant, are applied[2].

In agreement with the presence of several lipase genes in *Candida rugosa*[3,4], several lipase isoforms have been isolated from commercial enzyme preparations, where the gene product of *lip1* constitutes the major lipase form[5,6]. The expression of different genes coding for distinct isoforms seems to be regulated by the cultivation conditions (data not published). Notwithstanding the high homology among the cloned genes (60-70% sequence identity), differences in glycosylation of these lipases may also contribute to their heterogeneity[6].

Since the isoforms might differ in their catalytic performances[6] and some purification procedures affect the conformation of the lipases[7,8], the cloning and the separate expression of lipase genes seems to be the most suitable approach for the production and characterisation of pure isoforms and will also allow the production of mutant lipases with optimised properties for biocatalytic applications.

However, *Candida rugosa* is a dimorphic yeast in which, as in some other phylogenetically related *Candida* species[9], the triplet CUG, a universal codon for leucine, is read as serine[10]. This unusual amino acid assignment of the CUG codon relies on the presence in *C. rugosa* of an unusual $tRNA^{Ser}$ with an anticodon CAG[11]. In most *Candida* species, CUG is extremely rare, with the notable exception of *Candida rugosa* where it accounts for about 40% of the total serine codons[3'4]. Accordingly, multiple copies of the genes coding for $tRNA^{Ser}$ CAG have been isolated from the genome of this yeast[11].

As a consequence of this unusual codon-usage, the heterologous expression of *lip1* in *Saccharomyces cerevisiae* resulted in an inactive lipase[12]. Hence the exchange of the CUG codon by universal serine triplets is required for the expression of a functional protein in heterologous hosts. This was described by Zimmer and Schunck (1995)[9] for the heterologous expression of two other genes encoding cytochrome P450 cloned from another *Candida* microorganism, viz. *C. maltosa.* The expression of these genes in *S. cerevisiae,* however, resulted in the formation of still active but unstable enzymes and therefore cannot be considered a succesful route for the expression of industrially useful expression products. The occurrence of this affected phenotype is postulated to be related to the low frequency of CTG codon in these genes. In general, the CTG codon has a rather low frequency in the C. *maltosa* genome and has only been found so far in *P450alk* genes, *his5* and *ura3* among the C. *maltosa* genes sequenced.

On the contrary, in *Candida rugosa* lipases, CTG is used with a high frequency (3% of the codons), including that corresponding to the catalytic Ser 209 In the *lip1* gene (ORF 1647 nt), 20 out of 42 serine residues are encoded by CUG triplets[3]. There are 20 CUG serines: 19 in the mature protein and 1 in the leader sequence.

Alberghina and Lotti (1997)[13] disclose the expression of the *C. rugosa* lipase 1 gene in *Saccharomyces cerevisiae.* The original 15 residue leader sequence was replaced by the 16 residue leader sequence of the killer toxin of *Kluyveromyces lactis,* which resulted in high levels of mRNA and intracellular accumulation of lipase 1. Apparently, the *C. rugosa* lipase 1 expressed with the *K. lactis* leader peptide does not proceed along the secretory pathway but remains entrapped in an inactive form in the membranes probably due to a misfolding of the recombinant protein.

WO 99/14338 discloses nucleic acid sequences encoding a *C. rugosa* lipase, where at least 60%, preferably more than 80 or 90%, of the CTG codons encoding serine in *C. rugosa* have been replaced by a universal code for serine. These sequences are capable of expressing the lipase in a heterologous host cell, e.g. *Saccharomyces cerevisiae* or *Pichia pastoris,* and of secreting the lipase from the host cells in high yields, good purity, and in active form, thus enabling industrial large scale production. Typically, a supernatant comprising the lipase with contamination with

other proteins of less than 20% is obtained; a purity of more than 90% is often accomplished, and the resulting *C. rugosa* lipase is substantially free of other *C. rugosa* lipase isoforms.

As mentioned above, the *Candida rugosa* lipases currently known are useful and therefore commercially applied, in particular in the hydrolysis and synthesis of a wide range of esters of commercial interest. Nevertheless, there is a need for lipases with an altered substrate specificity, which are particularly suitable to hydrolyse a larger proportion of higher fatty acid esters from an oil, notably $C_{16}$-$C_{18}$ fatty acid esters, such as palmitates and stearates, to produce a margarine with lower saturated fatty acid content. Such a margarine would be advantageous in that it contributes in preventing diseases, such as arteriosclerosis[19, 20].

Furthermore, it would be useful to further improve the codon usage of the *Candida rugosa* lipase gene, so as to further increase the production of lipase, in particular by heterologous gene expression.

It is therefore an object of present invention to provide genetically modified nucleic acid sequences derived from the nucleic acid sequences disclosed in WO 99/14338, which are capable of producing active lipases with altered substrate specificity and/or in higher yield.

## Summary of the Invention

According to a first aspect of the present invention there is provided an amino acid sequence encoding a lipase, which has at least 65% sequence homology with the amino acid sequence of a *Candida ruginosa* lipase having the amino acid sequence substantially as depicted in Figure 2, and which differs by at least one amino acid substitution at a selected site in said lipase. Preferably, the sequence homology is at least 80%, and in particular more than 90%.

In a preferred embodiment the amino acid sequence according to the invention is at least substituted at one or more of the positions T11 end T201, preferably by alanine.

According to a second aspect of the invention there is provided a modified nucleic acid sequence encoding a lipase variant, said lipase variant being a variant of a ripening form of a *C. rugosa* lipase, said ripening form being selected from pre, pro, prepro or mature lipase, said nucleic acid sequence comprising 60% or less of the CTG codons at positions encoding serine as present in the corresponding native *C. rugosa* encoding sequence, said CTG codons having been replaced by a universal codon for serine, wherein that said modified nucleic acid sequence has been further modified such that said lipase variant exhibits an altered property. Preferably, said lipase variant is a variant of lipase 1. The lipase variant differs in at least one amino acid from the parent lipase.

Preferably, said modified nucleic acid sequence encodes a variant of lipase 1 wherein the modified residues are located at one or more of the positions T11 and T201 in the amino acid sequence of *Candida rugosa* lipase 1 as depicted in Figure 2, or a corresponding position in a different lipase. Preferably, the threonine at one or more of the positions 11 and 201 is replaced by alanine.

In a preferred embodiment, more than 70%, preferably more than 80%, most preferably more than 90% of the CTG codons at serine encoding positions of the starting lipase encoding nucleic acid sequence have been replaced.

In a further aspect of the invention lipase variants of a parent lipase are provided, wherein the parent lipase is encoded by an amino acid sequence substantially as depicted in Figure 1 and SEQ ID NO:2, and wherein the said amino acid sequence has been modified such that said lipase variants exhibit an altered property. Preferably, the parent lipase is obtainable from a strain of the genus *Candida,* most preferably of the species *Candida rugosa*.

In still another aspect of the invention expression vectors and modified host organisms are provided for expressing the modified nucleic acid sequences and secreting the amino acid sequences and/or lipase variants according to the invention. Preferred host organisms are for producing the amino acid sequences and/or lipase variants of the invention include eukaryote microorganisms, for example yeasts of the genus *Saccharomyces*, *Kluyveromyces*, *Hansenula* and *Pichia,* or fungi of the genus *Aspergillus* or *Trichoderma.*

In a further aspect of the invention methods are provided to prepare and select the modified nucleic acid sequences according to the invention and to produce the amino acid sequences and lipase variants of the invention.

In still a further aspect of the invention the use of the lipase variants and/or amino acid sequences is provided in a manner known per se, in particular in a process requiring high specificity towards $C_{16}$-$C_{18}$ acyl chains.

Also provided is the use of a modified nucleic acid encoding a lipase variant according to the invention, or part of said nucleotide sequence encoding a functional part of a lipase, as a probe for picking up a natural lipase by hybridisation.

These and other embodiments will be described in more detail in the description which follows.

## Brief Description of the Drawings

Figure 1 depicts the nucleotide sequence of a synthetic gene encoding *Candida rugosa* lipase 1, as described in WO 99/14338, as well as the corresponding amino acid sequence of lipase 1. Figure 2 shows the pYes2 vector, also referred to as I-pY-p-slip1, with restriction sites, the lipase gene (*lip*), and the ampicillin *(bla)* and uracil *(ura3)* genes.

Figure 3 gives a schematic representation of the error prone PCR strategy which is used in the present invention.

Figure 4 gives a schematic representation of the microtiter plate assay used in the present invention.

Figure 5 shows comparative results of the activity of three different lipases, the lipase from wild type *lip 1* and two mutants with one specific mutation, on a random oil with a fatty acid spectrum from $C_8$ to $C_{18}$.

**Definitions**

As used herein, an "enzyme variant" or a "modified enzyme", "mutated enzyme" or "mutant enzyme" is defined as an enzyme which closely resembles the corresponding naturally occurring enzyme, but which is different in one or more amino acids, e.g. by substitution, deletion or insertion of one or more amino acids. They will exhibit a high degree of homology (in terms of identity of residues) of at least 65%, preferably at least 80% or 90% or even 95% with the corresponding naturally occurring enzyme.

A "mutant gene" (other than one containing only silent mutations) means a gene encoding an enzyme having an amino acid sequence which has been derived directly or indirectly, and which at one or more locations is different from the sequence of a corresponding parent enzyme.

The "parent enzyme" means the gene product of the corresponding unaltered gene.

The term "altered property" is meant to indicate either altered substrate specificity or a higher production level as compared to the parent enzyme, or preferably both of these characteristics.

A "silent mutation" in a gene means a change or difference produced in the polynucleotide sequence of the gene which (owing to the redundancy in the codon-amino acid relationships) leads to no change in the amino acid sequence of the enzyme encoded by that gene.

A "mutant micro-organism" or "mutated micro-organism" means a micro-organism that is, or is descended from, a parent micro-organism subjected to mutation in respect of its gene for the enzyme. Such mutation of the organism may be carried out either (a) by mutation of a corresponding gene (parent gene) already present in the parent micro-organism, or (b) by the transfer (introduction) of a corresponding gene obtained directly or indirectly from another source, and then introduced (including the mutation of the gene) into the micro-organism which is to become the mutant micro-organism.

A "host organism" is an organism, usually a micro-organism (preferred), cell-line or plant cell, of which a mutant gene, or a transferred gene of other origin, forms part. In general it may be of the same or a different strain or species origin or descent as the parent organism.

"Homology" of two amino acid or nucleotide sequences is defined as the percentage of identical amino acids or nucleic acids, upon alignment of the two sequences according to sequence alignment software, such as ClustalW (Thompson, J.D., et al. Nucleic Acids Res. (1994) 22:4673-80.).

**Detailed Description of the Invention**

The invention relates in one embodiment to variants of lipase enzymes exhibiting an altered property, which term is defined herein as an altered substrate specificity or a higher production level as compared to the parent enzyme, or preferably both of these characteristics. A typical and preferred characteristic of altered substrate specificity according to the present invention is the capacity of hydrolyzing a larger proportion of higher fatty acid esters from an oil, notably $C_{16}$-$C_{18}$ fatty acid esters, such as palmitates and stearates. This altered substrate specificity exemplified above is also referred to as altered chain length specificity.

As a typical example of the present invention the lipase variants described herein are derived from a parent lipase which is obtainable from *Candida rugosa.* In this specification we have used predominantly a synthetic nucleic acid sequence encoding a *C. rugosa* lipase as a typical example of carrying out the methods according to the invention, said nucleic acid sequence being described in WO 99/14338. This known *Candida rugosa* lipase will be also referred to hereinafter as "lipase 1" or "lip 1", and the synthetic nucleic acid sequence encoding this lipase 1 will be also referred to as "slip 1". The amino acid sequence of this *C. rugosa* lipase 1 is depicted in Figure 1, using the sequence numbering of the vector and SEQ ID NO:2. The synthetic amino acid sequence encoding said *C. rugosa* lipase is also depicted in Figure 1 and in SEQ ID NO:1. As compared to the natural gene encoding the said *C. rugosa* lipase, the synthetic nucleic acid sequence differs in that all 19 CTG codons coding for serines, at positions 55, 59, 91, 93, 209, 241, 247, 282, 305, 348, 349, 365, 389, 407, 436, 440, 496, 498, and 531, have been replaced with TCT or TCC. As taught in WO 99/14338, the replacement of one or more CTG serine codons by universal serine codons such as TCT or TCC resulted in a better codon usage and hence a higher production yield of active lipase.

Thus, the synthetic nucleic acid sequence of Figure 1 was found a suitable starting point for investigating further mutations in the gene which would result in either an altered substrate activity of the lipase upon expression and secretion or a further improved codon usage with a concomitant higher yield of lipase, in original or modified form,

or both. However, any natural gene such as a gene encoding a lipase having an amino acid structure which is substantially depicted in Figure 1, or another gene disclosed in WO 99/14338 in which not all CTG codons have been substituted can be used as starting material for the desired further mutations, as indicated above. Alternative to the improvement of *Candida rugosa* lipase by modification of its gene, genetic information encoding lipases from other eukaryotic organisms can be isolated using 5'- and 3'- DNA probes derived from cDNA of other organisms encoding (pro)lipase, which will be fully evident to persons skilled in the art, and probes recognizing conserved sequences in other lipolytic enzymes and, if necessary, using these probes to multiply cDNA's derived from messenger RNA's (mRNA's) of lipase producing eukaryotic cells using the Polymerase Chain Reaction or PCR technology (see, for example WO-A-92/05249). Modifications of the lipase encoding gene in order to alter at least one property of the lipase as defined above can be accomplished in several ways, the principles of which are known in the art, such as site-directed or random mutagenesis followed by appropriate selection of the lipases thus obtained and, if desired, optimization. In a typical example on how the present invention can be practiced we will demonstrate the modification of chain length specificity of a lipase by altering the synthetic *lip1* gene by random mutagenesis 18, in particular error- prone PCR[16,17], followed by testing the resulting lipases using appropriate assays.

In this specification amino acids and amino acid residues in protein sequences are indicated by one-letter and three-letter abbreviations (Table 1). An amino acid present in the amino acid sequence of a protein is described by the position and the identity of the residue type, e.g. Ser59.

Table 1

| | |
|---|---|
| A = Ala = Alanine | V = Val = Valine |
| L = Leu = Leucine | I = Ile = Isoleucine |
| P = Pro = Proline | F = Phe = Phenylalanine |
| W = Trp = Tryptophan | M = Met = Methionine |
| G = Gly = Glycine | S = Ser = Serine |
| T = Thr = Threonine | C = Cys = Cysteine |
| Y = Tyr = Tyrosine | N = Asn = Asparagine |
| Q = Gln = Glutamine | D = Asp = Aspartic Acid |
| E = Glu = Glutamic Acid | K = Lys = Lysine |
| R = Arg = Arginine | H = His = Histidine |

In the present specification a numbering is based on the amino acid sequence of *Candida rugosa* lipase depicted in Figure 1 and SEQ ID NO:2.

<u>Plasmid</u>

Plasmid pYES2 (see Figure 2) is used as a suitable starting vector for insertion of the lipase gene. Plasmid pYES2 is a yeast expression vector and is designed for high level expression in *Saccharomyces cerevisiae.* This vector contains the *URA3* gene for selection in yeast and the ampicillin gene for selection in *E. coli.* The GAL1 promoter contains the sequences required for the regulation of transcription initiation as well as a mRNA starting site. Transcription may be induced only in the presence of galactose.

<u>Site directed mutagenesis</u>

Site directed mutagenesis is used to destroy one restriction site of *Bgl*I (1183) in the vector, because *Bgl*I and *BamH*I are used to cut the gene out of the vector.

<u>Error-prone PCR</u>

The error prone PCR is a PCR technique known in the art in which non-optimal reaction conditions are used with the idea that the *taq* polymerase does not work properly and makes mistakes[16,17]. The strategy for the error-prone PCR which we followed is schematically represented in Figure 3. Details will be given in the Experimental part.

After amplification of the *lip1* gene (1.6 kbp fragment) and the preparation of the vector (6 kbp) with digestion with *Bgl*I and *BamH*I the recombination of the fragment is carried out *in vivo* by transformation in *S. cerevisiae.*

Transformation in S. cerevisiae

Transformation of the modified lip1 gene is suitably carried out in *S. cerevisiae.* Several transformation protocols have been investigated and it turned out that the transformation in this yeast is preferably carried out with lithium acetate, as described by Gietz et al.

3,200 lipase variants were obtained which were transferred to microtiter plates.

Development of a microtiter plate assay

For an effective screening of altered chain-length specificity of the lipase variants we developed a microtiter plate assay involving a Biomek robot. Clearly, the assay has to be simple and quick. The principle of the assay is schematically shown in Figure 4 and the details will be given in the experimental part below.

Assay with p-nitro phenyl esters

For the screening of altered chain-length specificity the following p-nitrophenyl esters with different chain lengths were used:

1. *p*-nitrophenyl butyrate (pNPB)
2. *p*-nitrophenyl caprylate (pNPC)
3. *p*-nitrophenyl laurate (pNPL)
4. p-nitrophenyl palmitate (pNPP)
5. p-nitrophenyl eicosapentaenoate (pNPE)
6. p-nitrophenyl docosate (pNPD)

In the presence of a lipase the ester bond is hydrolysed and the produced p-nitrophenol (yellow) is detected at 410 nm. The intensity of absorption is a measure of the conversion and thus of the lipase activity.

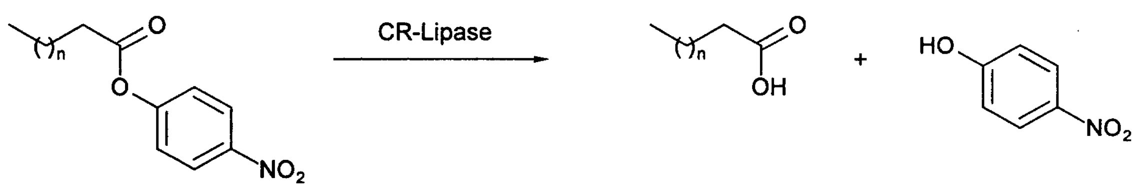


Screening of the mutants

All mutants were screened by using the described assay. Six clones showed a higher activity against special fatty acids in comparison to the wild type.

Sequencing of the clones

After isolation of the plasmids the clones were sequenced three times to prove the mutations.

Table 2

| Location of the mutations | |
|---|---|
| Clone | Mutations |
| MN9E4 | no mutations |
| MN9F3 | 1 silent mutation: leucine 515 TTG = leucine 515 CTG |
| MN17B11 | 1 mutation: threonine 11 ACC = alanine 11 GCC |

Table 2 (continued)

| Location of the mutations | |
|---|---|
| Clone | Mutations |
| MN17E2 | 2 silent mutations:<br>aspartate 191 GAC = aspartate 191 GAT<br>isoleucine 374 ATC = isoleucine 374 ATT |
| MN18H3 | 1 mutation: threonine 201 ACT = alanine 201 GCT |

Characterization of mutants

The mutants were cultivated in a 2 litre scale. The supernatant which contains the lipase, was separated by centrifugation. The lipase was concentrated by ultracentrifugation.

The activity of the mutants was investigated with a random oil with a fatty acid spectrum from $C_8$ to $C_{18}$. The fatty acid composition is in all positions very similar; see Table 3 below.

Table 3

| Fatty acid composition of the random oil | | |
|---|---|---|
| Fatty acid | Composition [mol%] | Composition 2-MG [mol%] |
| C 8:0 | 13,2 | 9,4 |
| C 10:0 | 8,9 | 10,0 |
| C 12:0 | 15,6 | 17,1 |
| C 14:0 | 5,5 | 5,8 |
| C 16:0 | 12,9 | 13,5 |
| C 18:0 | 10,8 | 11,3 |
| C 18:1 | 16,4 | 16,4 |
| C 18:2 | 14,6 | 14,3 |
| C 18:3 | 1,9 | 1,8 |

The mutant MN17B11 (threonine 11 was exchanged to alanine 11) showed a clear difference in activity against palmitic acid and stearic acid. It is three times more active against these fatty acids than the wild type.

It will be clear to the man skilled in the art that the approach described above results in a library of lipase variants in an expression host which can be screened for the appropriate enzyme activity.

The insight in the structure opens a variety of possibilities for developing novel enzymes with desired, tailor-made properties. In addition it provides insight in the enzymatic features that control substrate specificity and provides the opportunity to develop new improved lipases with a more specific or broader spectrum of activities than hitherto known. Any selected amino acid replacements can be engineered in the DNA encoding the lipase enzyme by methods well known to a person skilled in the art (e.g. site-directed mutagenesis). The DNA encoding the lipase or mutants thereof may be cloned on an expression vector and this construct may be transformed to a host wherein the gene is expressed, the mRNA translated and preferably the mature protein, or a precursor, secreted from the cell. Subsequently the protein can be purified. Standard procedures can be found in Sambrook et al.[24]

The application of these methods gives rise to a lipase variant, which is obtainable by the expression of a gene encoding said lipase encoding enzyme having an amino acid sequence which differs in at least one amino acid from the parent lipase enzyme. The variant thus obtained may contain a single mutation, but two or more mutations, usually up to ten mutations are also possible. Some of these mutants with two or more mutations, aimed at combining the desired properties, show that the properties are at least partially cumulative.

Useful mutants may also be made by combining any of the mutations or sets of mutations described in this specification. Besides, it is possible to combine useful mutations as disclosed herein with mutations at other sites, which may or may not cause a substantial change in the properties of the enzyme. Therefore, it is of course also possible to combine two or more mutants with different properties in one enzyme product or in the same process. Such combination may or may not have a synergistic effect.

The method according to the present invention is useful for the production, screening and selection of mutant lipolytic enzymes which are derived from naturally produced lipases. Such mutants are, for example, those encoded by a gene derived from a wild-type gene of a *Candida* strain.

It will be appreciated that by deletions or insertions of the amino acids in the lipase polypeptide chain, either created artificially by mutagenesis or naturally occurring in lipases substantially homologous to the *Candida rugosa* lipase of SEQ ID NO:2, the numbering of the amino acids may change. However, it is to be understood that positions homologous to amino acid positions of said lipase are intended to fall under the scope of the appended claims.

The method for selecting lipase variants according to the present invention (which includes the production and screening) comprises essentially the following steps:

(a) obtaining a DNA sequence encoding a lipase;
(b) mutating said sequence at one or more nucleotide positions, either by site-directed or by random mutagenesis;
(c) cloning the mutated DNA sequence into an expression vector so as to effect the expression of said mutated DNA sequence;
(d) transforming a host organism or cell with said vector;
(e) culturing said host organism or cell under suitable conditions for the production of said lipase variant;
(f) recovering said lipase variant, and in case of a screening procedure,
(g) identifying those lipase variants having altered substrate activity which make them particularly useful for certain applications, e.g. high specificity toward $C_{16}$-$C_{18}$ acyl chains.

Suitable host strains for production of mutant lipases include transformable microorganisms in which expression of the lipases can be achieved in sufficiently high amounts. Specifically, host strains of the same species or genus from which the lipase is derived, are suitable, in particular a *Candida* strain, preferably *C. rugosa,* or a mutant thereof having substantially the same properties. Also heterologous strains such as *Saccharomyces* (e.g. *S. cerevisiae*), *Kluyveromyces* (e.g. *K. lactis), Hansenula, Trichoderma* (e.g. *T. reesii)* and *Pichia* (e.g. *P. pastoris)* are among the preferred microorganisms from which suitable host strains can be selected. Other suitable host strains include those strains which are substantially incapable of producing extracellular proteolytic enzymes prior to the transformation with a mutant gene.

Expression signals include sequences of DNA regulating transcription and translation of the lipase gene. Proper vectors are capable of replicating at sufficiently high copy numbers in the host strain of choice or enable stable maintenance of the lipase gene in the host strain by chromosomal integration. The appropriate selection of host organisms, DNA regulating sequences, vectors etc. may be made routinely by a person skilled in the art using standard procedures, without inventive skill.

The lipase variant enzymes are produced by cultivating, under appropriate fermentation conditions, a transformed host strain comprising the desired mutant lipase gene or genes, and recovering the produced enzymes. Preferably, the lipases being expressed are secreted into the culture medium, which facilitates their recovery, or in the case of gram negative bacterial host strains into the periplasmic space. For secretion, a suitable amino-terminal signal sequence is employed, preferably the signal sequence encoded by the original gene if this is functional in the host strain of choice.

In an aspect of the invention a process is provided for producing a lipase variant as defined before which comprises the steps of fermentatively cultivating an rDNA modified host organism containing a gene made by rDNA technique which encodes the lipase variant, making a preparation of the lipase variant by separating the lipase variant produced by the host organism either from the fermentation broth, or by separating the cells of the host organism from the fermentation broth, disintegrating the separated cells and concentrating or part purifying the lipase either from said broth or from said cells by physical or chemical concentration or purification methods.

**Experimental protocol**

In the following part of this specification experimental details are specified of examples and assays which have been carried out to illustrate the invention. All techniques used for the manipulation and analysis of nucleic acid materials were performed essentially as described by Sambrook et al. (1989)[14], except when indicated otherwise. If certain items or details have not been mentioned herein specifically, they are usually considered as fully obvious and within the skill of a person skilled in the art. Reference may also be made to our WO 99/14338, which is incorporated herein by reference, as are any references cited herein.

***Materials***

Restriction enzymes and taq polymerase were obtained from MBI Fermentas (St. Leon-Rot, Germany). DNA Gel-Extraction Kit, Midi Plasmid Kit, Mini Plasmid Kit were obtained from Qiagen (Hilden, Germany). Peptone, yeast extract, and yeast nitrogen base were obtained from Difco (Augsburg, Germany). All reagents were of analytical grade, unless stated otherwise.

***Strains, plasmids and media***

*E. coli* DH5α (Invitrogen) was used as the host for plasmid amplification; *Saccharomyces cerevisiae* strain WCG4 was obtained from Institute for Biochemistry (Stuttgart, Germany) for transformation in *S. cerevisiae.*

The *E. coli* strain was grown at 37°C in Luria-bertani medium (LB) containing 100µg/ml ampicillin for selection of cloned transformants that contained vector pYES2.

*S. cerevisiae* strains were cultivated in YEPG-Medium: (1% yeast extract, 2% peptone, 2% glucose). For induction of lipase secretion 2% galactose instead of glucose was used.

The procedures of culturing the transformed S. cerevisiae cells, protein extraction and detection are known in the art. See, for example, Fusetti et al[12].

***Recombinant DNA techniques and DNA-sequencing***

Standard recombinant DNA methods were carried out according to the methods described by Sambrook *et al.* [14] and Ausubel *et al.* [15], unless stated otherwise. Sequencing was performed by the fluorescence-based dideoxy DNA cycle sequencing method. The Taq Dye Deoxy™ Cycle Sequencing Kit and the 373A DNA Sequencing System were purchased by Applied Biosystems and used according to the manufacturer's instructions.

***Quik-change for site-directed mutagenesis***

<u>*PCR for Quik-change*</u>

| Pipette-scheme: | | |
|---|---|---|
| | 10 µl | 10 x *Pfu* (buffer) |
| | 2 µl | DNA from Midi preparation (1:10) |
| | 2,5 µl | Primer 1 (5 pmol/µl) |
| | 2,5 µl | Primer 2 (5 pmol/µl) |
| | 2 µl | dNTP-Mix (2,5 mM each nucleotide) |
| | 82 µl | water |
| | 3 µl | *Pfu* Polymerase |

| Reaction programme for PCR: | | | |
|---|---|---|---|
| | 4 min | 95 °C | |
| | 1 min | 95 °C | |
| | 1,5 min | 54 °C | 20 x |
| | 16 min | 72 °C | |
| | 1 min | 95 °C | |
| | 1,5 min | 54 °C | |
| | 20 min | 72 °C | |
| | | 4 °C | |

The efficiency of the PCR reaction was checked by agarose gel electrophoresis. At 7.5 kbp there was one band which corresponds to the pYES + *lip1* vector. The whole sample was digested with *Dpn*I for 3 h at 37°C. The DNA was then transformed into XL2-Blue super competent cells.

***Digestion with BglI***

To check if restriction site (1183) is removed:

| | | |
|---|---|---|
| Pipette-scheme | 10 µl | DNA from Flexi preparation |
| | 2 µl | Puffer H |
| | 0,5 µl | *Bgl*I |
| | 7,5 µl | $H_2O$ |

The success of the site directed mutagenesis was controlled by gel electrophoresis.

***Digestion with BglI and BamHI***

| | | |
|---|---|---|
| Pipette-scheme | 20 µl | DNA from Midipreparation (pY-mutslip1) |
| | 10 µl | Puffer 2 |
| | 2 µl | *Bgl*I |
| | 3 µl | *BamH*I |
| | 1 µl | BSA |
| | 64 µl | $H_2O$ |
| | 100 µl | |

***Error prone PCR***

| | | |
|---|---|---|
| Pipette-scheme: | 2 µl | DNA from Midi preparation (pY-p-slip1)(Dilution 1:500) |
| | 1 µl | Primer 1 (50 pmol/µl) |
| | 1 µl | Primer 2 (50 pmol/µl) |
| | 8 µl | dNTP Mix |
| | 10 µl | *Taq* Puffer |
| | 8 µl | $MgCl_2$ |
| | 66 µl | $H_2O$ |
| | 4 µl | *Taq*-Polymerase |
| | 100 µl | |

| | | | |
|---|---|---|---|
| Reaction programme for error-prone PCR: | 4 min | 95 °C | |
| | 1,5 min | 95 °C | 25 x |
| | 1,5 min | 50 °C | |
| | 1,5 min | 72 °C | |
| | | 4 °C | |

***Agarose gel extraction***

The DNA from error prone PCR as well as from site-directed mutagenesis was separated by gel electrophoresis and purified by gel extraction.

The extraction was carried out with a DNA gel-extraction kit from Qiagen (Hilden, Germany).

### *Transformation*

Yeast cells were cultivated overnight at 30°C (OD = 1-2). 50 ml culture was transferred to a falcon tube and centrifuged 5 min at 3600 rpm. The pellet was washed with 50 ml sterile water, centrifuged again and washed a second time with 5 ml sterile water (centrifugation). The pellet was resuspended in 50 µl TE/LiAc solution, then 300 µl of PEG/TE/LiAc was added. The samples were incubated 30 min at 30°C, shaked at 42°C for 20 min. After the transformation the cells were cultivated on YNBaa plates.

YEPG-Medium: 1% yeast extract, 2% peptone, 2% glucose TE/LiAc solution: 1 ml TE (100 mM Tris, 10 mM EDTA), 1 ml LiAc (1 M), 8 ml sterile water

PEG/TE/LiAc solution: 8 ml PEG (50%), 1 ml TE, 1 ml LiAc YNBaa: 0.76% yeast nitrogen base (with 50 mg leucine + 50 mg histidine), 2% glucose, 1.5% agar)

### *Plasmid DNA Isolation*

The yeast clone with the plasmid was cultivated three days at 30°C in YEPG medium. The cultivation was centrifuged 5 min at 12,000 rpm. The pellet was treated with 200 µl of Breaking-buffer (2% Triton X-100, 1% SDS, 100 mM NaCl, 10 mM Tris/HCl pH 8, 1 mM EDTA), 200 µl phenol/chloroform and 300 mg glass pearls. This solution was mixed for 2 min with a vortex apparatus. After 5 min centrifugation, the water phase was used for transformation in *E. coli* (DH5α). Some colonies were picked from the plate and cultivated in LB (Luria-Bertani) medium. The DNA was isolated by midi preparation and the clones were sequenced three times.

### *Ultracentrifugation*

The lipase was concentrated by ultracentrifugation. A 10 KDa filter was used and the pressure was 4.7 bar.

### *Enzyme assays*

Lipase activity

The overall lipase activity was routinely measured by a pH-stat. Olive oil (5 ml) was emulsified with 100 ml water and 2 g gum Arabic using an Ultraturrax T25, Janke & Kunkel. 20 ml of this solution were transferred to a reaction vessel (30°C) and adjusted to pH 7. After addition of enzyme solution, the activity was measured with a pH-stat (Metrohm). One unit is defined as the release of 1µmol fatty acid/min.

Screening assay (see Figure 4)

After transformation the colonies were picked up and transferred to a microtiter plate containing glycerol (Stock plate). For the assay a replica plate (cultivation plate) was cultivated in a growing medium. In a new plate 120 µl buffer + 10 µl of p-nitrophenyl esters and 10 µl of lipase solution from cultivation plate was added. The absorption of the hydrolysed p-nitrophenol was detected at 410 nm.

The following esters were used:

1. *p*-nitrophenyl butyrate (pNPB)
2. *p*-nitrophenyl caprylate (pNPC)
3. *p*-nitrophenyl laurate (pNPL)
4. p-nitrophenyl palmitate (pNPP)
5. p-nitrophenyl eicosapentaenoate (pNPE)
6. p-nitrophenyl docosate (pNPD)

The above esters 1-4 are commercially available and esters 5 and 6 were synthesized chemically by methods known in the art.

### *Competitive assay for determination of chain length selectivity*

1 g random oil, 1 g gum Arabic and 20 ml water were mixed with an ultraturrax for 5 min. This solution (20

ml) was transferred into a reaction vessel. After addition of 470 µl $CaCl_2$ solution and lipase preparation, the separated fatty acids were titrated with 0.1 N NaOH (temperature: 30°C, hydrolysis: 10-15%). A sample of 2 ml was mixed with 85% phosphoric acid (200 µl), hexane + diethyl ether (1:1). The free fatty acids were collected in the hexane/diethyl ether phase, silylated and measured with the GC.

The fractions were combined and the solvent was carefully evaporated until a small residue. 10 µl of this solution was taken and completely evaporated. Then 10 µl of MSHFBA (Methyl silyl heptafluoro butyramide) was added and reacted for 15 min. After addition of 200 µl of dichloro methane, 1 µl was injected in GC and the free fatty acid were detected by FID (flame ionisation detector).

## Examples

### Blocking the tunnel: Engineering of Candida rugosa lipase mutants with short chain length specificity.

Based on X-ray structure, binding of fatty acids of different length to the scissile fatty acid binding tunnel of CRL were modelled. Residues which were assumed to mediate chain length specificity were replaced by more bulky amino acids, and the properties of resulting mutants were determined in different hydrolyzation assay.

### Methods

### Strains, plasmids and media

*E. coli* DH5α and Epicurian coli XL2-blue (Stratagene GmbH, Heidelberg, Germany) were used as hosts for plasmid amplification and *Pichia pastoris* GS115 (his4) (Invitrogen Corporation, San Diego, CA), for the expression of recombinant lipases using the vector pGAPZαB.

*E. coil* was grown at 37°C in low salt Luria-Bertani medium (LB) containing 25 µg/ml zeocin for selection of clones transformed with pGAPZαB derivatives˙ *P. pastoris* was grown in shaking flasks at 30°C in rich buffered medium containing 1 % glycerol and 0,1 M phosphate buffer pH 6 (BMGY, Invitrogen Manual *"Pichia* Expression Kit").

YPD medium (1 % yeast extract, 2 % peptone, 2 % glucose) was used for maintaining yeast cultures, and for selection of *P. pastoris* transformants YPD plates containing 2 % sorbitol or 1 % tributyrin and zeocin (100 µg/ml) were used.

### Construction of Lipl expression vector

The procedures for cloning the synthetic *lip1* (*slip1*) gene preceded by the *S. cerevisiae* prepro-α-factor leader sequence to give the plasmid pPIC-pp-slipl have been described previously (Brocca et al., 1998). To express the *slip1* gene under the constitutive GAP promoter, the expression vector pGAPZαB was used. The 1316 bp *Sfu*I-*Bam*HI/blunt fragment from plasmid pPIC-pp-*slip1*, containing a 5' truncated form of the *slip1* gene, was ligated into the *Sfu*I-*Xba*I/ blunt treated pGAPZ□B vector, giving plasmid pGAP-3'-*slip1*. Subsequently, in order to reconstitute the sequence encoding the mature lipase preceded by the α-factor prepro signal sequence, the 324 bp *SfuI* fragment from pPIC-pp-*slip1*, coding for the prepro-α-factor followed directly by the 5' part of the mature lipase, was inserted into *pGAP-3'-slip1* linearized with the same enzyme. The resulting plasmid pGAP-*slip1* was used as template for the site directed mutagenesis of *slip1*.

### DNA mutagenesis and sequencing

Site-directed mutagenesis was performed using the QuikChange™ Site Directed Mutagenesis Kit according to the manufacturers' instructions (Stratagene, Heidelberg, Germany), using 5-15 ng of *pGAP-slip1* DNA as template and couples of mutagenic, full length complementary oligonucleotides as primers. To simplify screening for mutant *slipl* genes, in some mutants diagnostic restriction sites as silent mutations were introduced by the mutagenic primers (Table I). Amplification products were treated for 2-3 hrs at 37°C with *Dpn*I endonuclease in order to remove traces of template DNA and directly used to transform supercompetent *Epicurian coli* cells according to Stratagene's instructions. For the construction of the double mutant L410F/S365L, the plasmid containing the mutation L410F, pGAP-*slip1-L410F,* was used as template with the appropriate primer pair in a second site-directed mutagenesis reaction. Mutant plasmid pGAP-*slip1*-L410F/S300E was obtained by ligation of a 0.7 kb *Spe*I/*Ssp*I fragment from plasmid pGAP-*slip1*-S300E into the 3.8 kb fragment derived from pGAP-*slip1*-L410F after digestion with the same enzymes.

The generated mutations were checked by restriction analysis using the introduced restriction sites and by automated sequencing. In order to avoid any mistake introduced by PCR into the whole plasmid, DNA fragments containing the-desired mutations were back-cloned into pGAP-*slip1* using standard procedures (Sambrook et al.,

1989). These plasmid reconstructions were checked by restriction analysis and by partial DNA sequencing around the junction sites. DNA sequencing was carried out using the BigDye™ Terminator Cycle Sequencing Ready Reaction Kit and a Abi Prism 377 DNA Sequencing System (Applied Biosystems) according to the manufacturer's instructions.

**Transformation of *P. pastoris* cells, plate assay of lipase activity and expression of Lip1**

*Pichia pastoris* GS115 cells were transformed by electroporation (Scorer et al., 1994) using a Bio-Rad Gene Pulser, with 10 µg of *Bsp*HI-linearized plasmid pGAP-*slip1* carrying wild-type or mutated *slip1* genes and plated onto YPDS plates containing zeocin. Positive transformants were checked for lipase activity by transferring colonies onto YPD plates containing 1 % of emulsified tributyrin and incubated o.n. at 30°C. Lipolytic activity was detected by the formation of a clear halo. 200 ml cultures of the wild-type or mutant *slip1* expressing clones were carried out in 1 l flasks at 30°C and 250 rpm in BMGY medium at pH 6.0 for 5 days. The cultures were maintained at constant pH by adding 1 M phosphate buffer pH 6.0 and fed by addition of 1 ml 86 % glycerol every 24 hours. After removing the cells by centrifugation, 0.1 % of sodium azide was added to the culture supernatants to prevent bacterial contamination. This was followed by filtration through cellulose nitrate (0.45 µm and 0.2 µm), and concentration of the supernatant to 5 % - 3 % of the original volume using Pall Filtron membrane type omega with a cut-off of 50 kDa. The concentrated lipases were used without further purification.

**Determination of lipase activity**

The lipolytic activity of the supernatants was routinely measured with tributyrin as substrate in a pH-stat assay at 30°C and pH 7.2 as described previously (Schmidt-Dannert et al. 1996). One unit (U) of lipase activity was defined as the amount of enzyme that liberates 1 µmol of fatty acids per minute under assay conditions. For determination of substrate specificity, triacetin, tricaproin and tricaprylin were used under the same conditions.

**Competitive assay for determination of chain length selectivity**

For the determination of chain length selectivity, a "randomized" triglyceride oil containing fatty acids of different chain lengths (saturated fatty acids C8:0 to C20:0, unsaturated fatty acids C18:1, C18:2, C18:3) randomly distributed over the sn-1, sn-2 and sn-3 position (Unilever). As the different chain lengths were not present in equal amounts within the randomized oil (from 0.4 to 16.4 mole%), the content of the different chain lengths was calculated in relation to oleic acid and the results were multiplied with the factor obtained for each fatty acid. The values presented are referred to as "standardized". 1 g (1.3 mmol) of randomized triglycerides were emulsified in 20 ml of distilled water containing 2 % (w/v) gum arabicum and used as substrate solution in a pH stat assay. 500 U of lipase (determined using tributyrin as substrate) were incubated at 30°C and pH 7.2. Liberated fatty acids were titrated automatically with 0.1 M NaOH. After 10 % and 20 % of hydrolysis, indicated by the amount of NaOH needed to maintain the pH, 2 ml of the reaction mixture were withdrawn, and the lipolytic reaction was stopped by the addition of 0.1 ml of 85 % orthophosphoric acid. As 1.3 mmol of triglycerides can liberate 4.9 mmol of free fatty acids, 10 percent of hydrolysis are reached after consumption of 0.4 mmol NaOH.

For gas chromatographic analysis, free fatty acids were extracted 3 times with diethylether:n-hexan (1:1), the extract was evaporated under nitrogen and resuspended in 0.5 ml of n-hexan. 0.1 ml of this solution were again evaporated under nitrogen and resuspended in 50 µl of MSHFBA (N-methyl-N-trimethylsilyl-heptafluorbutyramid) (Fureby et *al.,* Biocatal. Biotransf.**14;** 89-111, 1996) and incubated for 15 min at room temperature to convert the free fatty acids into the corresponding silyl-ethers before stopping the reaction by adding 100 µl of dichloromethan and analyzing 1 µl by GC (gas chromatograph: Fison 800, column: Optima 5 (25 m x 0.25 mm) (Macherey & Nagel), temperature program: 50°C, 5°C/min 300°C 2 Min, injector 370°C, flame ionisation detector 370°C, 100 KPa. A negative control, consisting of the substrate emulsion without lipase, was included for each measurement. As C18:3 and C20:0 were only present al 1.9 and 0.4 mole%, respectively, detection of the appropriate derivatives by GC was not possible.

**Modelling**

The structure of CRL in complex with inhibitor hexadecanesulfonate was retrieved from the Protein Databank [Bernstein], PDB entry 1LP0. The inhibitor binds inside a tunnel which is flanked by mostly hydrophobic residues. Since it is assumed that the scissile fatty acid binds similarly, the position of a fatty acid chain was estimated by superposing C1 of the fatty acid with the sulfur atom of the inhibitor, C2 of the fatty acid with C1 of the inhibitor and so forth. Thus, the inhibitor mimics binding of a C17 fatty acid chain. Residues were considered to be mutated if (1) located at the inner wall of the tunnel, (2) their side chains pointed towards the hexadecanesulfonate.

## Results

### Characterisation of mutants

To determine the substrate specificity of the CRL mutants for fatty acids of short and medium chain length, concentrated supernatants of recombinant *Pichia pastoris* cultures expressing CRL (wild-type) or the appropriate lipase mutant were used in a pH stat assay at 30°C and pH 7.2 using triacetin (C2), tributyrin (C4), tricaproin (C6) and tricaprylin (C8) as substrates. Neither the wild-type nor the lipase mutants hydrolyzed triacetin in substantial amounts. The relative activity of CRL wild-type and the mutants toward tributyrin, tricaproin and tricaprylin is shown in Fig. 6, except for mutant L304F which showed no activity for the three substrates under these assay conditions. The activity toward tricaprylin (C8) was set as 100 %. Mutant S300E behaves nearly identical to the wild-type, whereas mutants L413F, L410W, L410F/S300E and L410F/S365L are similar with a slightly higher hydrolytic activity toward tricaproin (C6) and an up to 30% lower activity toward tributyrin. However, mutant P246F differs substantially, as it has a very high activity on tributyrin and tricaproin compared to the wild-type.

### (1) Blocking near the entrance at fatty acid position C4 (L304F, L413F, Fig.7a)

Two hydrophobic leucines which are positioned near C4 of the scissile fatty acid are replaced by bulky, hydrophobic phenylalanines. Since the side chains point toward the tunnel, the phenylalanine side chains are expected to accommodate without disturbing the protein structure.
The chain length profile of the two mutants is similar: In comparison to the wild-type the percentage of hydrolysis for C8 is about 2-3 fold higher for both mutants, but very similar for C10. In contrast, the release of fatty acids with a chain length longer than C12 is decreased for both mutants. Taking into account the different reaction times to reach 10 % of hydrolysis, position L304 seems to be more critical to lipase activity.

### (2) Blocking at fatty acid position C6/C8 (P246F, Fig. 7b)

The small and hydrophobic proline is replaced by the more bulky, hydrophobic phenylalanine. Since the $C_\alpha$ -$C_\beta$ bond of P246 points toward tunnel, the mutation should not influence binding of scissile fatty acids shorter than C6.
Blocking at C6/C8 reduced activity toward medium and long chain fatty acids as expected, although also for this mutant, the reaction times were much longer. The hydrolysis of C8 for the mutant is about 4 fold higher than for the wild-type, whereas a discrimination against long chain fatty acids is observable.

### (3) Blocking at C16/C18 (L410W, L410F/S300E, L410F/S365L, Fig 7c)

The medium sized, hydrophobic L410 is replaced by the most bulky, slightly hydrophilic tryptophan. In addition, two double mutants were investigated, with L410 replaced by phenylalanine combined to a mutation of a serine near the C20 position of the fatty acid.
As expected, for all three mutants chain lengths of C16 and longer are only poor substrates.The profile was most pronounced for mutant L410W, as its activity toward C14 is also strongly decreased.

### (4) Blocking the exit at fatty acid position C18/C20 (S300E, Fig. 7d)

S300 is located near the surface of CRL and was replaced by the bulky, hydrophilic glutamic acid.
The chain length profile of mutant S300E and wild-type are similar, with a slight decrease of selectivity toward long chain fatty acids.

### References

1. Woolley,P.& Petersen, S.B. 1994 *Lipases* Cambridge University Press, Cambridge, UK
2. Weber, H.K., Stecher, H. and Faber, K. 1995 Some properties of commercially available crude lipase preparations. *Preparative Biotransformations.* Robert, S.M. Ed p.5-21
3. Longhi, S., Fusetti, F, Grandori, R., Lotti, M., Vanoni, M. and Alberghina, L. 1992. Cloning and nucleotide sequences of two Lip genes from *Candida cylindracea.. Biochim.Biophys.Acta.* **1131:**227-232.
4. Lotti, M., Grandori, R., Fusetti, F., Longhi, S., Brocca, S., Tramontano, A., Alberghina, L. 1993. Molecular cloning and analysis of Candida cylindracea lipase sequences. *Gene,* **124:**45-55

5. Chang, R.C., Chou, S.J., Shaw, S.F. 1994 *Biotechnol. Appl. Biochem.* **19**:93-97

6. Rua M.L., Diaz-Maurino T., Fernandez V.M., Otero C., Ballesteros A. 1993. Purification and characterisation of two distinct lipases from *Candida cylindracea*. *Biochim. Biophys. Acta* **1156:**181-189

7. Wu, S.H., Guo, Z.W., Sih, C.J. 1990. *J.Am.Chem. Soc.* **112**:1990-1995

8. Colton, I.J., Ahmed, S.H., Kazlauskas,R.J. 1995. *J.Org.Chem.* **60:**212-217

9. Zimmer, T., Schunck W.H 1995. A deviation from the universal genetic code in *Candida maltosa* and consequences for the heterologous expression of cytochromes P45052A4 and 52A5 in *Saccharomyces cerevisiae. Yeast* **11:**33-41

10. Kawaguchi, Y., Honda, H., Taniguchi-Morimura, H., Iwasaki, S. 1989. The codon CUG is read as serine in an asporogenic yeast *Candida cylindracea. Nature* **341:**164-166

11. Yokogawa, T., Suzuki, T., Ueda, T., Mori, M., Ohama, T., Kuchino, Y., Yoshinari S., Motoki, I., Nishikawa, K., Osawa, S., Watanabe, K. 1992. Serine tRNA complementary to the non universal serine codon CUG in *Candida cylindracea:* evolutionary implications. *Proc.Natl.Acad.Sci.USA.* **89:**7408-7411.

12. Fusetti, F., Brocca, S., Porro, D., Lotti, M., 1996. Effect of the leader sequence on the expression of recombinant *C.rugosa* lipase by *S.cerevisiae* cells *Biotechnology Letters*, **18**:281-286

13. Alberghina L. and Lotti M. (1997) Journal of molecular catalysis B3, pag 37-41

14. Sambrook J., Fritsch, E.F., and Maniatis, T. 1989. Molecular Cloning: a laboratory Manual, Second Edition (Plaiview, New York: Cold Spring Harbor Laboratory Press)

15. Ausubel, F.M., Brent, R., Kingston, R.E., Moore, D.D., Seidman, J.G., Smith, J.A., and Struhl, K 1994. *Current Protocols in Molecular Biology* VolI. New York: Greene Publishing Associates and Wiley-Interscience

16. Cadwell R.C. and Joyce G.F.; Mutagenic PCR, *PCR Methods Appl.* 1994 **3**(6):136-140

17. Cadwell R.C. and Joyce G.F.; Randomization of genes by PCR mutagenesis. *PCR Methods Appl.* 1992 **2**(1): 28-33

18. Papworth C., Braman J. and Wright D.A. (1996) *Strategies* **9**(1):3-4

19. Williams C.M., *et al.* Cholesterol reduction using manufactured foods in high monosaturated fatty acids: a randomized crossover study. *Br J Nutr.* (1999) **81**(6):439-446

20. Salo P., *et al.* Effect of low fat, low-cholesterol dietary intervention on fatty acid compositions in serum lipid fractions in 5-year old children. The STRIP project. *Eur J Clin Nutr.* (*1999*) **53**(12):927-932.

## Claims

**1.** An amino acid sequence encoding a lipase, **characterised in that** it has at least 65% sequence homology with the amino acid seqence of a *Candida rugosa* lipase having the amino acid sequence substantially as depicted in Figure 1 and SEQ ID NO:2, and which differs by at least one amino acid substitution at a selected site or at a non-selected site by random mutagenisis in said lipase.

**2.** An amino acid sequence according to claim 1, wherein said sequence homology is at least 80% and preferably more than 90%.

**3.** An amino acid sequence according to claim 1 or 2, wherein one or more of the following positions of the *Candida rugosa* lipase having substantially the amino acid sequence as depicted in Figure 1 and SEQ ID NO:2, or a corresponding position in a different lipase, is substituted: T11, T201.

**4.** An amino acid sequence according to any one of claims 1 to 3, wherein the threonine at one or more of the positions 11 and 201, or a corresponding position in a different lipase, is substituted by alanine.

**5.** A modified nucleic acid sequence encoding a lipase variant, said lipase variant being a variant of a ripening form of a *C. rugosa* lipase, said ripening form being selected from pre, pro, prepro or mature lipase, said nucleic acid sequence comprising 60% or less of the CTG codons at positions encoding serine as present in the corresponding native *C. rugosa* encoding sequence, said CTG codons having been replaced by a universal codon for serine, **characterised in that** said modified nucleic acid sequence has been further modified such that said lipase variant exhibits an altered property.

**6.** A modified nucleic acid sequence of claim 5, wherein said lipase variant is a variant of lipase 1.

**7.** A modified nucleic acid sequence of claim 5 or 6, wherein said lipase variant differs in at least one amino acid from the parent lipase.

**8.** A modified nucleic acid sequence of any one of claims 5 to 7, wherein said modified nucleic acid sequence encodes a variant of lipase 1 wherein the modified residues are located at one or more of the following positions in the

amino acid sequence of *Candida rugosa* lipase 1 as depicted in Figure 1 and SEQ ID NO:2, or a corresponding position in a different lipase: T11, T201.

**9.** A modified nucleic acid sequence of claim 8, wherein said modified nucleic acid sequence encodes a variant of lipase 1, in which the threonine at one or more of the positions 11 and 201 is replaced by alanine.

**10.** A modified nucleic acid sequence of any one of claims 5 to 9, wherein more than 70%, preferably more than 80%, most preferably more than 90% of the CTG codons at serine encoding positions have been replaced.

**11.** A lipase variant of a parent lipase, said parent lipase being encoded by an amino acid substantially as depicted in Figure 2, **characterised in that** said amino acid sequence has been modified such that said lipase variant exhibits an altered property.

**12.** A lipase variant as claimed in claim 11, **characterised in that** it is encoded by a modified nucleic acid sequence as claimed in any one of claims 5 to 10.

**13.** A lipase variant as claimed in claim 11 or 12, wherein the parent lipase is obtainable from a strain of the genus *Candida,* and preferably of the species *Candida rugosa.*

**14.** An expression vector comprising a modified nucleic acid sequence as claimed in any one of claims 5 to 10, wherein said modified nucleic acid is operably linked to a promoter.

**15.** An rDNA modified host organism which has been transformed by a DNA vector carrying a modified nucleic acid sequence encoding a lipase variant as claimed in any of claims 11 to 13 and which is capable of expressing said lipase variant.

**16.** An rDNA modified host organism according to claim 15, said host organism further being capable of secreting protein.

**17.** An rDNA modified host organism according to claim 15 or 16 carrying a gene encoding a lipase variant that is introduced into the host organism by fusion at its 5'-end to a gene fragment encoding a (modified) pre-sequence functional as a signal- or secretion-sequence for the host organism.

**18.** An rDNA modified host organism according to any one of claims 15 to 17, wherein said host organism is a eukaryote microorganism selected from the group consisting of the genus *Saccharomyces, Kluyveromyces, Hansenula* or *Pichia,* or a fungus of the genus *Aspergillus* or *Trichoderma.*

**19.** An rDNA modified host organism comprising one or more of a modified nucleic acid sequence as claimed in any one of claims 5 to 10, or an expression vector as claimed in claim 14 comprising one or more of said modified nucleic acid sequence.

**20.** A process for producing a lipase variant as defined in any one of the preceding claims, which comprises the steps of fermentatively cultivating an rDNA modified host organism containing a gene made by rDNA technique which encodes said lipase variant, making a preparation of said lipase variant by separating said lipase variant produced by said rDNA host organism either from the fermentation broth, or by separating the cells of the host organism from the fermentation broth, disintegrating the separated cells and concentrating or purifying the lipase either from said broth or from said cells by physical or chemical concentration or purification methods.

**21.** A process for obtaining a lipase variant molecule as claimed in any one of claims 11 to 13 which comprises the following steps:

(a) obtaining a DNA sequence encoding a lipase;
(b) mutating said sequence at one or more selected nucleotide positions or by performing a random mutagenisis;
(c) cloning the mutated DNA sequence into an expression vector so as to effect the expression of said mutated DNA sequence;
(d) transforming a host organism or cell with said vector;
(e) culturing said host organism or cell under suitable conditions for the production of said lipase variant;

(f) recovering said lipase variant.

**22.** An enzymatic composition comprising a lipase variant as claimed in any one of claims 11 to 13 or an amino acid sequence as claimed in any one of claims 1-4.

**23.** Use of the lipase variant as claimed in any one of claims 11 to 13 or of an amino acid sequence as claimed in any one of claims 1 to 4, in a manner known per se in a process requiring high specificity towards $C_{16}$-$C_{18}$ acyl chains.

**24.** Use of a nucleotide sequence as claimed in any one of claims 1 to 4, or of a nucleotide sequence encoding a lipase variant as claimed in any one of claims 11 to 13, or part of said nucleotide sequence encoding a functional part of a lipase, as a probe for picking up a natural lipase by hybridisation.

## Figure 1

**Sequence of the synthetic lip1-gene from Candida rugosa**

```
5450  cgg atc gga cta cta gca gct gta ata cga ctc act ata ggg aat att aag

5501  ctt ttg att tta acg act ttt aac gac aac ttg aga aga tca aaa aac aac

5552  taa tta ttc gaa acg atg aga ttt cct tca att ttt act gct gtt tta ttc

                                      A   P   T   A   T   L   A   N   G   D
5603  gca gca tcc tcc gca tta gct|gcc cca acc gcc act ttg gct aac ggt gac

      T   I   T   G   L   N   A   I   I   N   E   A   F   L   G   I   P
5654  acc atc acc ggt ttg aac gcc atc atc aac gaa gcc ttc ttg ggt att cca

      F   A   E   P   P   V   G   N   L   R   F   K   E   P   V   Q   Y
5705  ttt gcc gaa cca cca gtt ggt aac ttg aga ttc aag gac cca gtt cca tac

      S   G   S   L   D   G   Q   K   F   T   S   Y   G   P   S   C   M
5756  tcc ggt tcc ttg gat ggt caa aag ttc act tct tac ggt cca tct tgt atg

      Q   Q   N   P   E   G   T   Y   E   E   N   L   P   K   A   A   L
5807  caa caa aac cca gaa ggt acc tac gaa gaa aac ttg cca aag gca gct tta

      D   L   V   M   Q   S   K   V   F   E   A   V   S   P   S   S   E
5858  gat ctg gtt atg caa tcc aaa gtt ttc gaa gct gtt tct cca tct tct gaa

      D   C   L   T   I   N   V   V   R   P   P   G   T   K   A   G   A
5909  gac tgt ttg acc att aat gtt gtt aga cca ccc ggg aca aag gct ggt gcc

      N   L   P   V   M   L   W   T   F   G   G   G   F   E   T   G   G
5960  aac ttg cca gtt atg ttg tgg atc ttt ggt ggt ggt ttt gaa gtt ggt ggt

      T   R   T   F   P   P   A   Q   M   I   T   K   S   I   A   M   G
6011  act agt acc ttc cct cca gcc caa atg att acc aag tct att gct atg ggt

      K   P   I   I   H   V   S   V   N   Y   R   V   S   S   W   G   F
6062  aag cca atc atc cac gtt tct gtc aac tac aga gtc tcg agc tgg ggt ttc

      L   A   G   D   E   I   K   A   E   G   S   A   N   A   G   L   K
6113  ttg gct ggt gac gaa atc aag gcc gaa ggt tct gcc aac gcc ggt ttg aag

      D   Q   R   L   G   M   Q   W   V   A   D   N   I   A   A   F   G
6164  gac caa aga ttg ggt atg caa tgg gtg gct gac aac att gct gct ttt ggt
```

## Fig. 1 (cont'd)

```
      G   D   P   T   K   V   T   I   F   G   E   S   T   T   S   M   S
6215  ggt gat cca act aag gtt act atc ttt ggt gaa tct gct ggt tct atg tcc

      V   M   C   H   I   L   W   N   D   G   D   N   T   Y   K   G   K
6266  gtc atg tgt cac att ttg tgg aac gac ggt gac aac act tac aag ggt aag

      P   L   F   R   A   G   I   M   Q   S   G   A   M   V   P   S   D
6317  cca ttg ttc aga gct ggt atc atg caa tct ggt gct atg gtt cca tct gac

      A   V   D   G   I   Y   G   N   E   I   F   D   L   L   A   S   N
6368  gcc gtc gac ggt atc tac ggt aac gaa att ttt gac ttg ttg gct tcc aac

      A   G   C   G   S   A   S   D   K   L   A   C   L   R   G   V   S
6419  gct ggt tgt ggt tct gcc tct gac aag ttg gct tgt ttg aga ggt gtt tct

      S   E   T   L   E   D   A   T   N   N   T   P   G   F   L   A   Y
6470  tct gac act ttg gaa gac gcc acc aac aac acc cct ggt ttc ttg gct tac

      S   S   L   R   L   S   Y   L   P   R   P   D   G   V   T   I   T
6521  tcc tcc tta aga ttg tct tac ttg cca aga cca gac ggt gtt aac atc acc

      D   D   M   Y   A   L   V   R   E   G   K   Y   A   N   I   P   V
6572  gac gac atg tac gct ttg gtt aga gaa ggt aag tat gcc aac atc cct gtt

      I   I   G   D   Q   N   D   E   G   T   F   F   G   T   S   S   L
6623  atc atc ggt gac caa aac gac gaa ggt acc ttc ttt ggt act tct tct ttg

      N   V   T   T   D   A   Q   A   R   E   Y   F   K   Q   S   F   V
6674  aac gtt acc act gat gcc caa gcc aga gaa tat ttc aag caa tct ttt gtc

      H   A   S   D   T   E   I   D   T   L   M   T   A   Y   P   G   D
6725  cac gct agc gac gct gaa atc gac act ttg atg act gct tac cca ggt gac

      I   T   Q   G   S   P   F   D   T   G   I   L   N   A   L   T   P
6776  atc act caa ggt tct cca ttt gac act gga att cta aac gcc ttg acc cca

      Q   F   K   R   I   S   A   V   L   G   D   L   G   F   T   L   A
6827  caa ttc aag aga atc tct gct gtt ttg ggt gac ttg ggt ttt act ttg gct

      R   R   Y   F   L   N   H   Y   T   G   G   T   K   Y   S   F   L
6878  cgt aga tac ttc ttg aac cac tac acc ggt ggt acc aag tac tct ttc ttg

      S   K   Q   L   S   G   L   P   V   L   G   T   F   H   S   N   D
6929  tct aag caa ttg tct ggt ttg cca gtt ttg ggt act ttc cac tcc aac gat
```

**Fig. 1 (cont'd)**

```
      I   V   F   Q   D   Y   L   L   G   S   G   S   L   I   Y   N   N
6980  atc gtc ttc caa gac tac ttg ttg ggt tct ggt tcc ttg atc tac aac aac

      A   F   I   A   F   A   T   D   L   D   P   N   T   A   G   L   L
7031  gct ttc att gct ttt gcc act gac ttg gac cca aac acc gcc ggt ttg ttg

      V   K   W   P   E   Y   T   S   S   S   Q   S   G   N   N   G   M
7082  gtt aag tgg cca gaa tac acc tct tct tct caa tct ggt aac aac ttg atg

      M   I   N   A   L   G   L   Y   T   G   K   D   N   F   R   T   A
7133  atg atc aac gct ttg ggt ttg tac acc ggt aag gac aac ttc aga acc gcc

      G   Y   D   A   L   F   F   N   P   P   S   F   F   V
7184  ggt tac gac gct ttg ttc tcc aac cca cca tct ttc ttt gtt| tga taa taa

7235  ggc ctg gat cca cta gta acg gcc gcc agt gtg ctg gaa ttc tgc aga tat

7286  cca tca cac tgg cgg ccg ctc gag cat gca tct aga ggg ccg cat cat gta

7337  att agt tat gtc acg ctt aca ttc acg ccc tcc ccc cac atc cgc tct aac

7388  cga aaa gga agg
```

| = start and end of the lipase gene

<u>Primers for error-prone PCR</u>:

| | | |
|---|---|---|
| Primer mutCRL1 | 5' cggatcggactactagcagc 3' | (SEQ ID NO: 3) |
| Primer mutCRL2 | 3' cggttagagcggatgtggg 5' | (SEQ ID NO: 4) |

<u>Sequence primers</u>:

| | | |
|---|---|---|
| Primer PYES 2 | gcagctgtaatacgactc (5' → 3') | (SEQ ID NO: 5) |
| Primer C | ttagaccacccgggacaaaggctgg (5' → 3') | (SEQ ID NO: 6) |
| Primer D | atctgacgccgtcgacggtatctacgg (5' → 3') | (SEQ ID NO: 7) |
| Primer PYES 1 | cgtgaatgtaagcgtgac (3' → 5') | (SEQ ID NO: 8) |

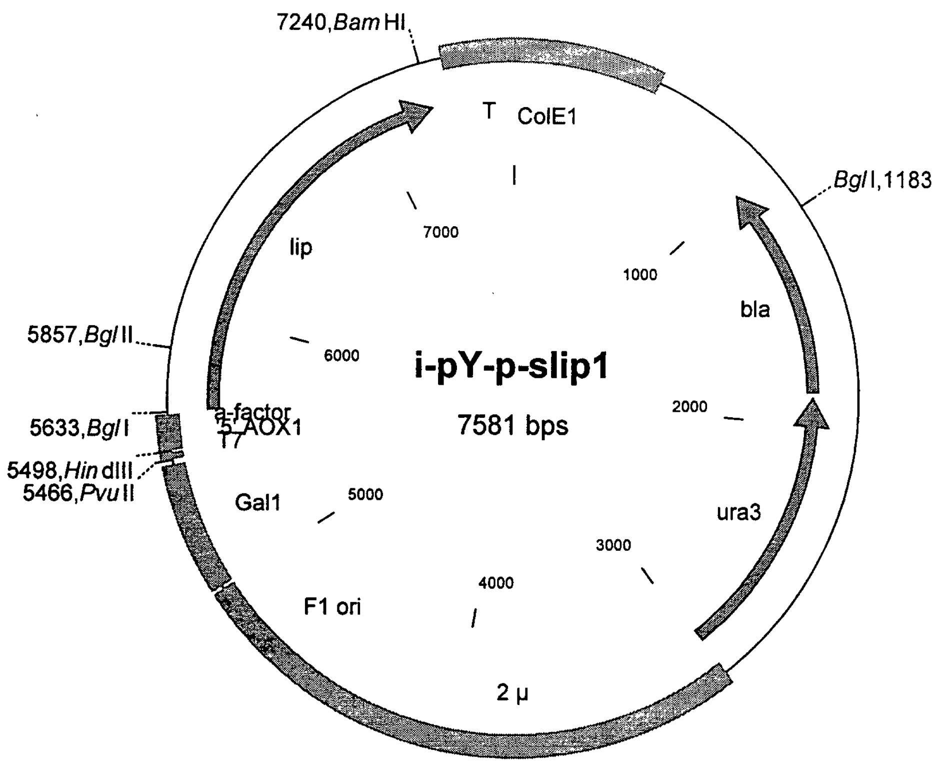
7240,BamHI
T
ColE1
BglI,1183
7000
lip
1000
bla
5857,BglII
6000
i-pY-p-slip1
7581 bps
2000
5633,BglI
α-factor
5'AOX1
T7
5498,HindIII
5466,PvuII
Gal1
5000
ura3
3000
4000
F1 ori
2 µ

Fig. 2

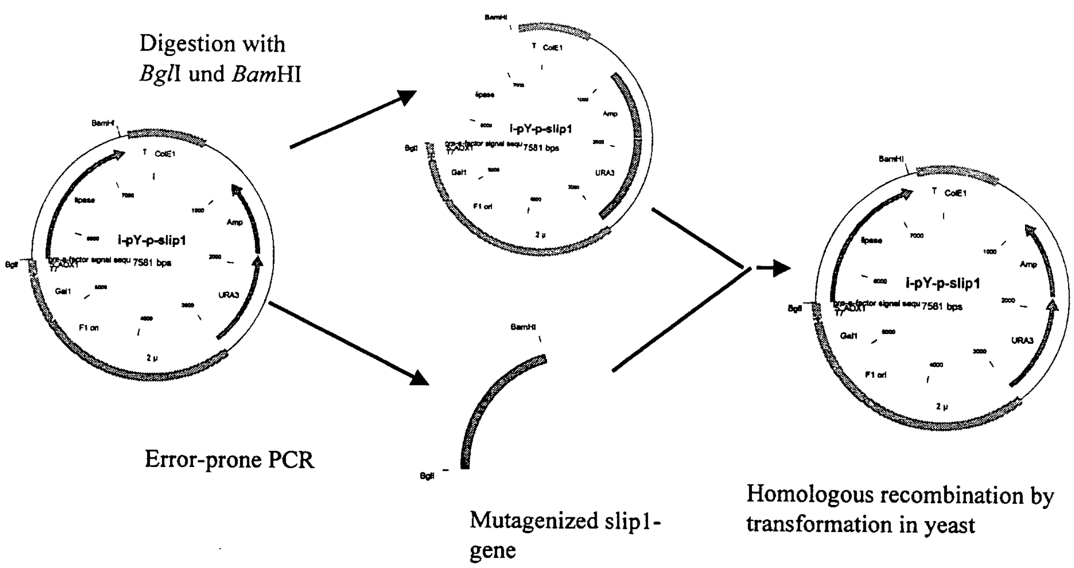
Digestion with
*Bgl*I und *Bam*HI
Error-prone PCR
i-pY-p-slip1
7581 bps
Mutagenized slip1-
gene
Homologous recombination by
transformation in yeast

**Fig. 3**

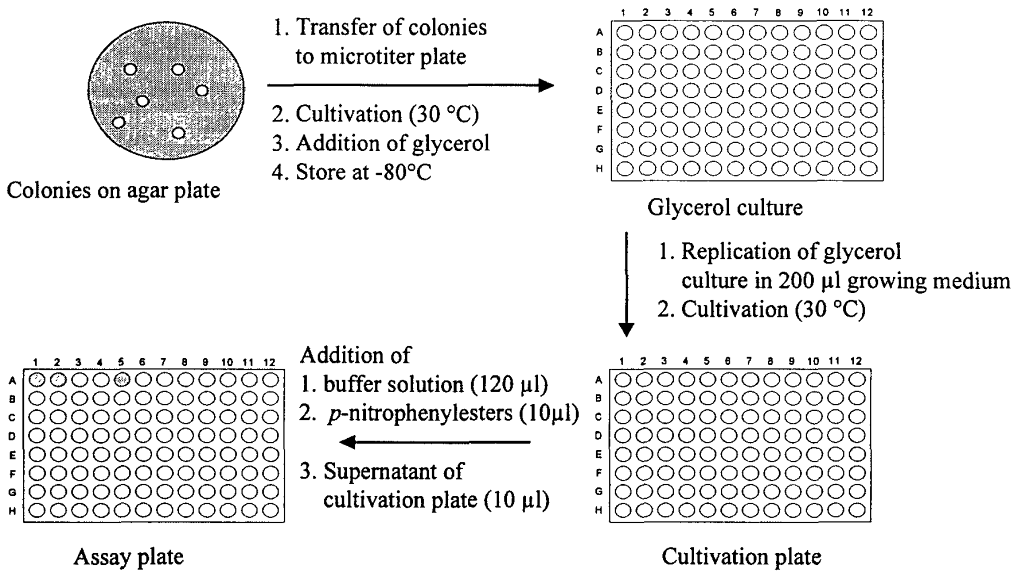
1. Transfer of colonies
to microtiter plate
2. Cultivation (30 °C)
3. Addition of glycerol
4. Store at -80°C
Colonies on agar plate
Glycerol culture
1. Replication of glycerol
culture in 200 µl growing medium
2. Cultivation (30 °C)
Addition of
1. buffer solution (120 µl)
2. *p*-nitrophenylesters (10µl)
3. Supernatant of
cultivation plate (10 µl)
Assay plate
Cultivation plate

**Fig. 4**

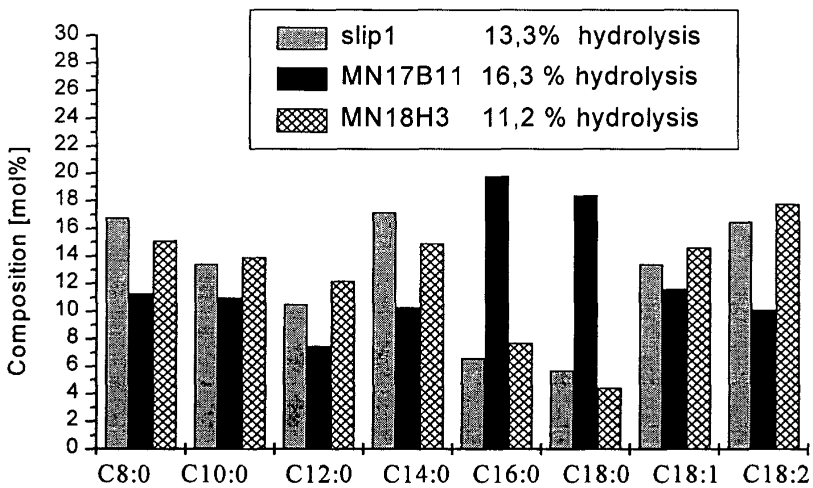
slip1 13,3% hydrolysis
MN17B11 16,3 % hydrolysis
MN18H3 11,2 % hydrolysis
Composition [mol%]
30
28
26
24
22
20
18
16
14
12
10
8
6
4
2
0
C8:0
C10:0
C12:0
C14:0
C16:0
C18:0
C18:1
C18:2

**Fig. 5**

**Figure 6: Activity of CRL mutants toward short chain triacylglycerides.**

Relative activities were determined using pH stat assay at 30°C and pH 7.2 using the amount of lipase corresponding to 500 U measured with tributyrine. The activity toward tricaprylin was set as 100 %. Mutant L304F did not show any activity using these substrates.

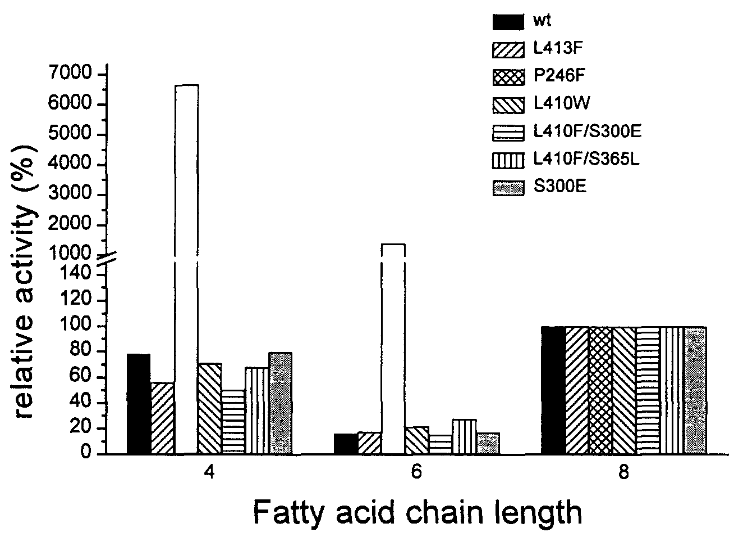

**Figure 7: Substrate specificity of CRL mutants using a "randomized" oil.**

Hydrolysis of the "randomized" oil was carried out in a pH stat at 30°C and pH 7.2 until titration of 0.4 mM free fatty acids (10 % of hydrolysis). After derivatization of the fatty acids to the corresponding silyl ether, the samples were analyzed by GC analysis. The standardized values were calculated as described in the material and methods section.

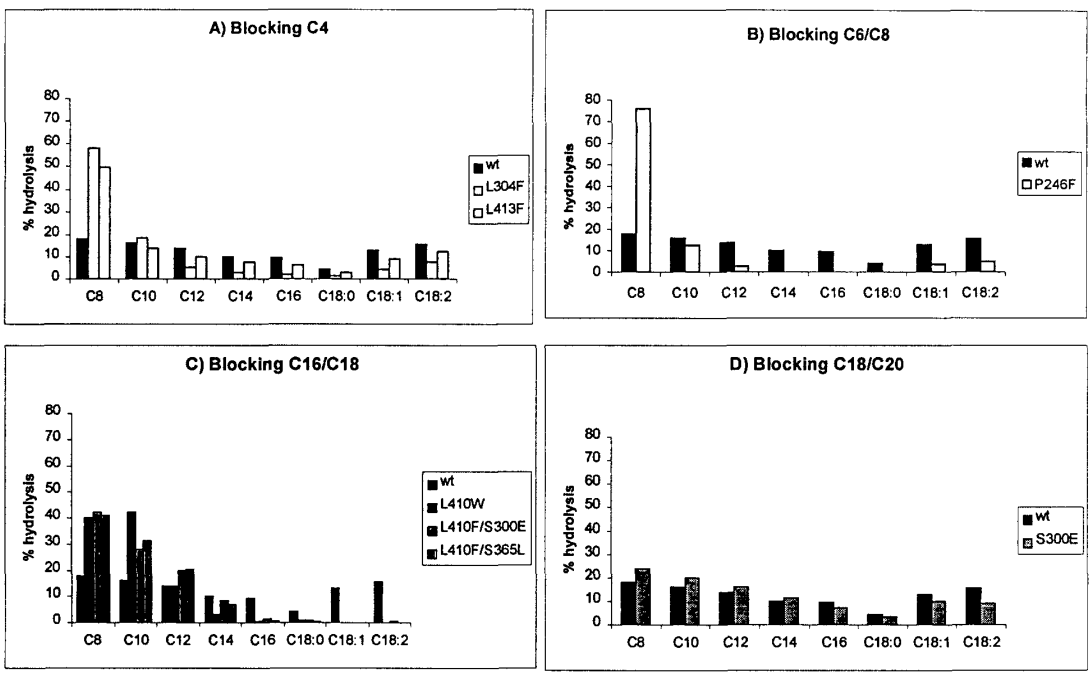

European Patent Office

# EUROPEAN SEARCH REPORT

Application Number
EP 01 20 0375

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | WO 99 14338 A (SCHMIDT DANNERT CLAUDIA ;SCHMID ROLF (DE); BROCCA STEFANIA (IT); L) 25 March 1999 (1999-03-25)<br>* page 6 *<br>* page 8, line 22 - line 30 *<br>* page 12, line 27 - line 29 *<br>--- | 1,2,5-7, 10-24 | C12N15/55<br>C12N9/20<br>C12Q1/68 |
| A | ZANDONELLA G ET AL: "INVERSION OF LIPASE STEREOSPECIFICITY FOR FLUOROGENIC ALKYLDIACYL GLYCEROLS EFFECT OF SUBSTRATE SOLUBILIZATION"<br>EUROPEAN JOURNAL OF BIOCHEMISTRY,DE,BERLIN,<br>vol. 231, no. 1, 1 July 1995 (1995-07-01), pages 50-55, XP000565709<br>ISSN: 0014-2956<br>* abstract *<br>----- | 1-13 | |
| | | | TECHNICAL FIELDS SEARCHED (Int.Cl.7) |
| | | | C12N<br>C12P |

The present search report has been drawn up for all claims

| Place of search | Date of completion of the search | Examiner |
|---|---|---|
| THE HAGUE | 27 March 2001 | Mata Vicente, T. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 01 20 0375

This annex lists the patent family membersrelating to the patent documents cited in the above-mentioned European search report.
The members are as contained in the European Patent Office EDP file on
The European Patent Office is in no way liable for these particulars which are merely given for the purpose of information.

27-03-2001

| Patent document cited in search report | Publication date | Patent family member(s) | Publication date |
|---|---|---|---|
| WO 9914338 A | 25-03-1999 | AU 4224997 A | 05-04-1999 |
| | | EP 1012301 A | 28-06-2000 |

EPO FORM P0459

For more details about this annex : see Official Journal of the European Patent Office, No. 12/82